(19) **Europäisches Patentamt European Patent Office Office européen des brevets**

(11) **EP 4 502 141 A1**

(12) **EUROPEAN PATENT APPLICATION**
published in accordance with Art. 153(4) EPC

(43) Date of publication:
**05.02.2025 Bulletin 2025/06**

(21) Application number: **23780149.3**

(22) Date of filing: **24.03.2023**

(51) International Patent Classification (IPC):
**C12N 5/00** *(2006.01)*    **C12M 1/00** *(2006.01)*
**C12M 3/00** *(2006.01)*

(52) Cooperative Patent Classification (CPC):
**C12M 1/00; C12M 3/00; C12N 5/00**

(86) International application number:
**PCT/JP2023/011777**

(87) International publication number:
**WO 2023/190139 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.03.2022   JP 2022056628**

(71) Applicant: **FUJIFILM Corporation
Tokyo 106-8620 (JP)**

(72) Inventors:
• **NAGASE, Masaya
  Ashigarakami-gun, Kanagawa 258-8577 (JP)**
• **TAKEMORI, Sho
  Ashigarakami-gun, Kanagawa 258-8577 (JP)**

(74) Representative: **Klunker IP
Patentanwälte PartG mbB
Destouchesstraße 68
80796 München (DE)**

(54) **CONSTRUCTION METHOD, CONSTRUCTION DEVICE, AND CONSTRUCTION PROGRAM**

(57)    An aspect of the present invention provides a configuration method, a configuration apparatus, and a configuration program that enable configuration of a culture medium panel having high suitability for cells having various characteristics under a size constraint of a limited culture medium set. A configuration method according to an aspect of the present invention is a configuration method for configuring a culture medium panel constituted by a plurality of culture media to optimize a cell culture result. The configuration method includes, by a processor, a region setting step of setting a region of a culture medium composition space; a characteristic listing step of listing a plurality of cell characteristics; a prediction step of making a prediction of a culture result of a target cell for a given culture medium composition, based on a cell characteristic of the target cell; a search listing step of searching for and listing, for respective cell characteristics included in the plurality of cell characteristics, culture medium compositions for which cell culture results have local maximum values; and a configuration step of configuring the culture medium panel from culture medium composition candidates corresponding to the local maximum values.

FIG. 1

**Description**

**BACKGROUND OF THE INVENTION**

1. Field of the Invention

The present invention relates to a configuration method, a configuration apparatus, and a configuration program for configuring a culture medium panel constituted by a plurality of culture media.

2. Description of the Related Art

Recently, with the spread of gene editing technology and so on, diversification of various cells such as CHO cells (Chinese Hamster Ovary cells) for producing antibody drugs, HEK cells (Human Embryonic Kidney cells) for producing viruses for gene therapy, and iPS cells (Induced Pluripotent Stem cells) for regenerative medicine has progressed. In general, in many cases, culture conditions of cells are optimized to increase the proliferative property of the cells or the yields of various products, and in particular, in many cases, it is desirable to optimize a culture medium for a specific cell.
For example, JP2014-503220A describes that a functional enviromics map representing the intensity of activation or repression of elementary cellular functions is constructed and that the functional enviromics map is used to develop an optimized cell culture medium formulation (culture medium composition). For example, WO2021/049044A describes manufacturing of a (new) culture medium based on prediction parameters of an object (cell) of culture, an evaluation index of the culture, and a culture medium manufacturing condition.

**SUMMARY OF THE INVENTION**

However, it takes time and cost to develop a culture medium dedicated to a certain cell (hereinafter referred to as a dedicated culture medium). In contrast, a method of selecting an appropriate culture medium by a small number of repeated experiments provides simple development of a dedicated culture medium and has limitations. In addition, a method of using some appropriate culture medium as a temporary measure has a problem in that it is not known how reliable the results are.
Accordingly, there is conceivable a method in which a "set" of a plurality of appropriate culture media (hereinafter referred to as a "culture medium panel") is configured in advance, a culture experiment is performed only once (or a few times) on the culture medium panel, and a culture medium for which any culture result is satisfactory is selected. In this case, even when a dedicated culture medium is to be developed, a culture medium included in a culture medium panel is used as the starting point for the development, thereby making it possible to reduce the duration and cost.
Accordingly, it is an important challenge to "configure a culture medium panel that can support a variety of cells".
Typical solutions to such a problem may include a method of configuring a panel by appropriately adding a proven culture medium on the basis of experience and findings obtained from previous culture experiments or the like. However, such typical solutions have the following difficult limitations: (1) since cell information cannot be obtained in advance, it is difficult to assume a kind of cell; however, diversification of various cells has progressed, as described above; and (2) since confirmation involves experimentation, the size of a culture medium panel (the number of culture media included in a culture medium set) cannot be so large. Accordingly, with progress in the diversification of cell types, the development of new technologies is required, including alternative methods for cases such as when a culture medium that was effective in the past fails to function, for example. However, such a situation has been difficult for conventional techniques such as those disclosed in JP2014-503220A and WO2021/049044A to address.
The present invention has been made in view of such circumstances, and an object thereof is to provide a configuration method, a configuration apparatus, and a configuration program that enable configuration of a culture medium panel having a small size and high suitability for cells having various characteristics.
To achieve the object described above, a configuration method according to a first aspect of the present invention is a configuration method for configuring a culture medium panel constituted by a plurality of culture media to optimize a cell culture result. The configuration method includes, by a processor, a region setting step of setting a region of a culture medium composition space; a characteristic listing step of listing a plurality of cell characteristics; a prediction step of making a prediction of a culture result of a target cell for a given culture medium composition, based on a cell characteristic of the target cell; a search listing step of searching for and listing, for respective cell characteristics included in the plurality of cell characteristics, culture medium compositions for which cell culture results have local maximum values; and a configuration step of configuring the culture medium panel from culture medium composition candidates corresponding to the local maximum values. In the search listing step, the processor extracts a culture medium composition candidate from the region of the culture medium composition space, predicts, for the extracted culture medium composition candidate, a culture result of a cell having a cell characteristic included in the plurality of cell characteristics in the prediction step, and

searches for and determines, from the predicted culture result, culture medium composition candidates that provide the local maximum values.

According to the first aspect, culture medium compositions that provide local maximum values of cell culture results are searched for and listed for respective cell characteristics included in a plurality of cell characteristics, and a culture medium panel is configured from culture medium composition candidates corresponding to the local maximum values of the culture results. This enables configuration of a culture medium panel having a small size (the number of culture media included in the culture medium panel being small) and high suitability for cells having various characteristics.

In the first aspect and the following aspects, the term "target cell" means a "cell given a specific cell characteristic". The "target cell" needs to have clearly defined characteristics, but is not necessarily "known" in the sense of being widely used or having been prepared, and a "new" cell to be prepared, that is, a cell that has not yet been prepared, and a "virtual cell having a virtual cell characteristic" are also included in the "target cell". However, an "unknown cell" in the sense that its characteristics are unclear is not included in the target cell.

In the first aspect and the following aspects, furthermore, the term "culture medium having a given composition" means a "culture medium having a specific composition" or a "culture medium having any composition", and the amounts of components of each composition are clearly defined. The term "culture medium having a given composition" is not limited to being "known" in the sense of being commonly used or having already been prepared, and may be a "new" culture medium to be prepared, that is, a culture medium that has not yet been prepared. However, an "unknown" culture medium with unclear amounts of components is not included in the "culture medium having a given composition".

In the first aspect and the following aspects, a culture result having a "local maximum value" means that "a culture result preferably has a value as close to the local maximum value as possible in consideration of individual cell characteristics, but may have a value that is not exactly the local maximum value but rather in the vicinity of the local maximum value in consideration of suitability for a plurality of cells".

A configuration method according to a second aspect is the configuration method according to the first aspect, in which in the configuration step, the processor configures the culture medium panel such that a distribution of the culture medium composition candidates in the culture medium composition space is widened. Selecting culture media constituting the culture medium panel from among various culture media makes it possible to support even an unknown cell having a characteristic slightly deviating from an expected cell characteristic, and the second aspect defines a specific aspect of such "various culture media".

A configuration method according to a third aspect is the configuration method according to the second aspect, in which in the configuration step, the processor configures the culture medium panel such that a correlation between the culture medium composition candidates in the culture medium composition space decreases, the culture medium panel such that a distance between the culture medium composition candidates in the culture medium composition space increases, or the culture medium panel such that diversity defined by an information gain of the culture medium composition candidates in the culture medium composition space increases. The third aspect defines an aspect of a method for configuring "the culture medium panel such that a distribution of the culture medium composition candidates in the culture medium composition space is widened" according to the second aspect.

A configuration method according to a fourth aspect is the configuration method according to any one of the first to third aspects, in which in the configuration step, the processor weights the culture medium composition candidates, based on a frequency of appearance of a cell characteristic, and configures the culture medium panel, based on a result of weighting of the culture medium composition candidates. According to the fourth aspect, an appropriate culture medium panel can be configured based on the frequency of appearance of a cell characteristic.

A configuration method according to a fifth aspect is the configuration method according to any one of the first to fourth aspects, including, by the processor, an evaluation step of evaluating a characteristic difference between cells; and a generation step of generating, based on the evaluated characteristic difference, virtual cell characteristics whose differences from a cell characteristic of a known cell group satisfy a constraint condition, while controlling the virtual cell characteristics, in which the plurality of cell characteristics to be listed in the characteristic listing step includes one or more of the generated virtual cell characteristics. As defined in the fifth aspect, in the present invention, when a plurality of cell characteristics are to be listed, virtual cell characteristics may be generated and added to known cell characteristics. In the fifth aspect, the term "known cell (group)" refers to cells that currently exist and are available. This manes that, since it is meaningless to consider an impossible cell characteristic, virtual cell characteristics are generated in consideration of a cell characteristic of the "known cell (group)" that currently exists.

A configuration method according to a sixth aspect is the configuration method according to the fifth aspect, in which in the generation step, the processor sets the constraint condition, based on a characteristic difference between cells in the known cell group. The sixth aspect defines an aspect of constraint condition setting.

A configuration method according to a seventh aspect is the configuration method according to any one of the first to sixth aspects, in which the processor segments the culture medium panel into subsets and associates at least one or more known cell lineages with any one of the subsets. When a culture medium panel associated with all expected cell lineages is to be configured, the culture medium panel may have a large size or culture medium compositions to be set may be coarse (culture medium compositions for which culture results have local maximum values or are in the vicinity thereof may be difficult to fully cover (may be difficult to include in the culture medium panel)). In this case, configuring the culture medium panel in the way as in the seventh aspect enables configuration of a culture medium panel having a more dense association (a culture medium panel configured from culture medium composition candidates fully covering culture medium compositions for which culture results have local maximum values or are in the vicinity thereof) while reducing the size of the panel.

A configuration method according to an eighth aspect is the configuration method according to the seventh aspect, in which the processor segments the culture medium panel into the subsets such that the subsets include a subset independent of a specific cell lineage. The eighth aspect defines an aspect of configuration of the subsets.

A configuration method according to a ninth aspect is the configuration method according to any one of the first to eighth aspects, in which in the configuration step, the processor configures the culture medium panel by using a submodular optimization method. The ninth aspect defines an aspect of a method for configuring a culture medium panel. "Submodular optimization" is an optimization method using the submodularity of a set function. "Submodularity" is a concept corresponding to convexity or concavity (in a continuous function) defined for a set, and is known to be applicable to combinatorial optimization.

A configuration method according to a tenth aspect is the configuration method according to any one of the first to ninth aspects, in which the processor uses a cell simulation including a metabolic pathway model to express at least one of a means for the prediction in the prediction step, a prediction function of an unknown cell, or a difference between cell characteristics. The tenth aspect specifically defines a method for predicting a culture result.

A configuration method according to an eleventh aspect is the configuration method according to any one of the first to tenth aspects, in which in the prediction step, the processor makes a prediction of the culture result by predicting a biological behavior amount of the target cell, based on the given culture medium composition and a culture condition; changing a cell environment of the target cell, based on a prediction result of the biological behavior amount; and repeating a prediction of the biological behavior amount and a change of the cell environment, based on the changed cell environment. The eleventh aspect specifically defines a method for predicting a culture result.

A configuration method according to a twelfth aspect is the configuration method according to the eleventh aspect, in which the processor performs the prediction of, as the biological behavior amount, a change in a total number of cells over time, a change in a substance produced by the target cell and an amount of the substance over time, and a change in a substance included in a culture environment of the target cell and an amount of the substance over time, and performs the change of, as the cell environment, a total number of cells of the target cell, the substance produced by the target cell, and an amount of the substance. The twelfth aspect further specifically defines the prediction according to the eleventh aspect.

A configuration method according to a thirteenth aspect is the configuration method according to any one of the first to twelfth aspects, in which the target cell is any one of a CHO cell, an HEK cell, or an iPS cell. The thirteenth aspect specifically defines an example of the target cell.

A configuration method according to a fourteenth aspect is the configuration method according to any one of the first to thirteenth aspects, in which the culture result is one or more of an antibody yield or a virus yield by the target cell, a proliferative property of the target cell, and a success rate of inducing differentiation of the target cell into a specific tissue. The fourteenth aspect defines an example of the culture result. In the present invention, it is preferable to use a culture result corresponding to the type of the target cell.

To achieve the object described above, a configuration apparatus according to a fifteenth aspect of the present invention is a configuration apparatus for configuring a culture medium panel constituted by a plurality of culture media to optimize a cell culture result. The configuration apparatus includes a processor configured to perform a setting process to set a region of a culture medium composition space; a listing process to list a plurality of cell characteristics; a prediction process to make a prediction of a culture result of a target cell for a given culture medium composition, based on a cell characteristic of the target cell; a search listing process to search for and list, for respective cell characteristics included in the plurality of cell characteristics, culture medium compositions for which cell culture results have local maximum values; and a configuration process to configure the culture medium panel from culture medium composition candidates corresponding to the local maximum values. In the search listing process, the processor is configured to extract a culture medium composition candidate from the region of the culture medium composition space, predict, for the extracted culture medium composition candidate, a culture result of a cell having a cell characteristic included in the plurality of cell characteristics in the prediction process, and search for and determine, from the predicted culture result, culture medium composition candidates that provide the local maximum values.

According to the fifteenth aspect, as in the first aspect, it is possible to configure a culture medium panel having a small size and high suitability for cells having various characteristics. The configuration apparatus according to the fifteenth aspect may include a configuration similar to those according to the second to fourteenth aspects.

To achieve the object described above, a configuration program according to a sixteenth aspect of the present invention is a configuration program for causing a processor to execute a configuration method for configuring a culture medium panel constituted by a plurality of culture media to optimize a cell culture result. The configuration method includes a region setting step of setting a region of a culture medium composition space; a characteristic listing step of listing a plurality of cell characteristics; a prediction step of making a prediction of a culture result of a target cell for a given culture medium composition, based on a cell characteristic of the target cell; a search listing step of searching for and listing, for respective cell characteristics included in the plurality of cell characteristics, culture medium compositions for which cell culture results have local maximum values; and a configuration step of configuring the culture medium panel from culture medium composition candidates corresponding to the local maximum values. The search listing step extracts a culture medium composition candidate from the region of the culture medium composition space, predicts, for the extracted culture medium composition candidate, a culture result of a cell having a cell characteristic included in the plurality of cell characteristics in the prediction step, and searches for and determines, from the predicted culture result, culture medium composition candidates that provide the local maximum values.

According to the sixteenth aspect, as in the first and fourteenth aspects, it is possible to configure a culture medium panel having a small size and high suitability for cells having various characteristics. The program according to the sixteenth aspect may include a configuration similar to those according to the second to fourteenth aspects. An aspect of the present invention can also provide a non-transitory tangible recording medium (e.g., various magneto-optical recording devices or semiconductor memories) storing computer-readable code of the program according to these aspects. The term "non-transitory tangible recording medium", as described above, does not include a non-tangible recording medium such as a carrier signal or a propagation signal itself.

As described above, a configuration method, a configuration apparatus, and a configuration program according to the present invention enable configuration of a culture medium panel having a small size and high suitability for cells having various characteristics.

## BRIEF DESCRIPTION OF THE DRAWINGS

Fig. 1 is a diagram schematically illustrating how a culture medium panel is a configured;
Fig. 2 is a diagram illustrating a configuration of a culture medium panel configuration system;
Fig. 3 is a block diagram illustrating a configuration of a processing unit;
Fig. 4 is a block diagram illustrating a configuration of a culture result prediction unit;
Fig. 5 is a diagram illustrating data stored in a storage unit;
Fig. 6 is a flowchart illustrating processing of a configuration method and a configuration program for configuring a culture medium panel;
Fig. 7 is a flowchart illustrating a culture result prediction method;
Fig. 8 is a diagram illustrating an overview of a simulator for a biological behavior amount prediction step;
Fig. 9 is a schematic diagram illustrating an example of cellular metabolic pathways;
Fig. 10 is a diagram illustrating an overview of the Michaelis-Menten equation;
Fig. 11 is a diagram illustrating an overview of existing cell metabolism models;
Fig. 12 is a schematic diagram illustrating creation of a cell death model by using machine learning;
Fig. 13 is a diagram conceptually illustrating a technique for configuring a culture medium panel;
Fig. 14 is a diagram illustrating a phylogenetic tree of CHO cells;
Fig. 15 is a diagram illustrating how a culture medium panel is segmented into subsets;
Fig. 16 is a schematic diagram illustrating a search effect obtained by simulation; and
Fig. 17 is a table illustrating results of comparison between a conventional method and methods according to embodiments of the present invention.

## DESCRIPTION OF THE PREFERRED EMBODIMENTS

Embodiments of a configuration method, a configuration apparatus, and a configuration program according to the present invention will be described in detail. In the description, reference is made to the accompanying drawings as necessary.

Overview of Process

The following describes an example of a search for a composition of a culture medium mainly for antibody-producing CHO cells, with the productivity to be optimized being the antibody yield thereof. However, the cell to be targeted in the present invention may be any one of a CHO cell, an HEK cell for producing a virus for gene therapy, and an iPS cell for regenerative medicine, and the intended productivity may also be one or more of the proliferative property of the cell, the antibody yield or the virus yield of the cell, and a success rate of inducing differentiation of the target cell into a specific tissue. Even when such target cells and productivities are selected, embodiments and examples having the same gist as those described below can be configured. The culture method can also be selected as appropriate from among well-plate (microtiter plate or microplate) culture, flask culture, batch culture, fed-batch culture, perfusion culture, and the like.

As used herein, the term "culture medium" refers to a mixture of nutrients for culturing cells, and the components thereof typically include, but are not limited to, amino acids, vitamins, metals, and so on. Accordingly, a culture medium can be represented as a multidimensional vector (or a point in a multidimensional space (hereinafter referred to as a "culture medium composition space") having dimensions corresponding to the respective nutrients). The present invention can optimize a partial or entire amount or a content ratio of the composition (nutrients) of such a culture medium. The culture medium composition space is typically multidimensional (the number of dimensions differs depending on the number of nutrients), but may be represented by one dimension in the following description for simplicity.

For example, the term "culture medium" refers to a CHO cell culture medium (CHO: Chinese Hamster Ovary), the term "cell" refers to a certain CHO cell (including not an only original CHO cell but also CHO cells of various lineages caused by mutation, modification, or the like, such as CHO-DG44, CHO-K1, and CHO-S), and the term "culture result" refers to the antibody yield obtained after culture of cells for a predetermined period of time. As described above, however, the culture medium, the cell, and the culture result in the present invention are not limited to these examples.

First Embodiment

Culture Medium Panel Configuration System

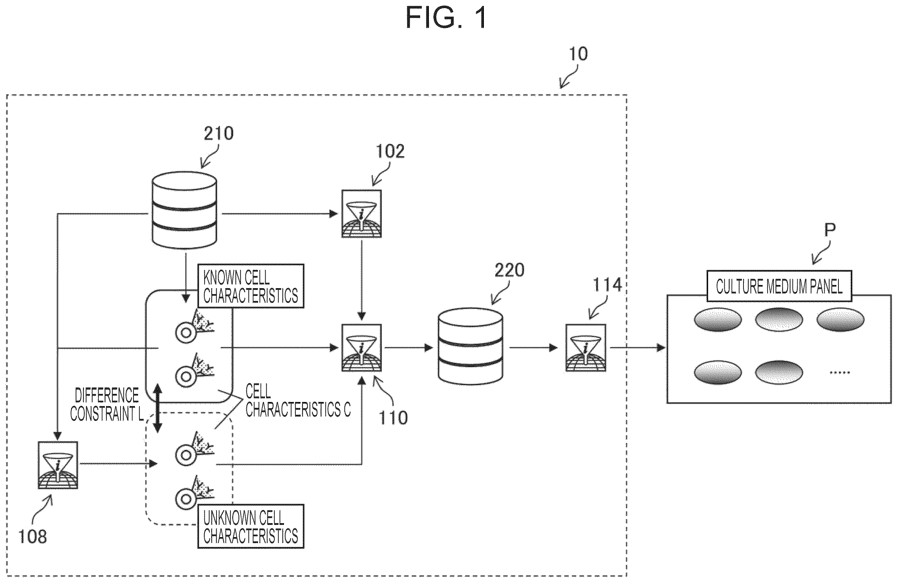

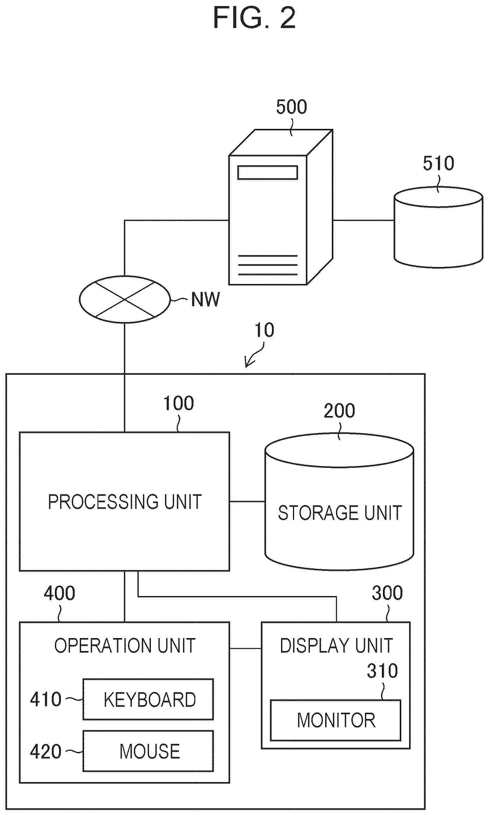

Fig. 1 is a diagram schematically illustrating how a culture medium panel is configured by a culture medium panel configuration system 10 (configuration apparatus) according to a first embodiment. Fig. 2 is a diagram illustrating a configuration of the culture medium panel configuration system 10. In the following, the description of some of the components of the culture medium panel configuration system 10 will be omitted as appropriate.

The culture medium panel configuration system 10 is a system for configuring a culture medium panel constituted by a plurality of culture media to optimize a cell culture result, and can be implemented using a computer. As illustrated in Fig. 2, the culture medium panel configuration system 10 includes a processing unit 100 (processor), a storage unit 200, a display unit 300, and an operation unit 400, which are connected to each other to transmit and receive necessary information. These components can be installed in various ways. The components may be installed in one location (such as in one housing or one room) or may be installed in separate locations and connected to each other via a network. The culture medium panel configuration system 10 can be connected to an external server 500 and an external database 510 via a network NW, such as the Internet, to acquire information on cell characteristics, culture medium compositions, cell lines, and so on, as necessary, and can provide information on an obtained culture medium panel and so on to a desired server or database, such as the external server 500 or the external database.

Configuration of Processing Unit

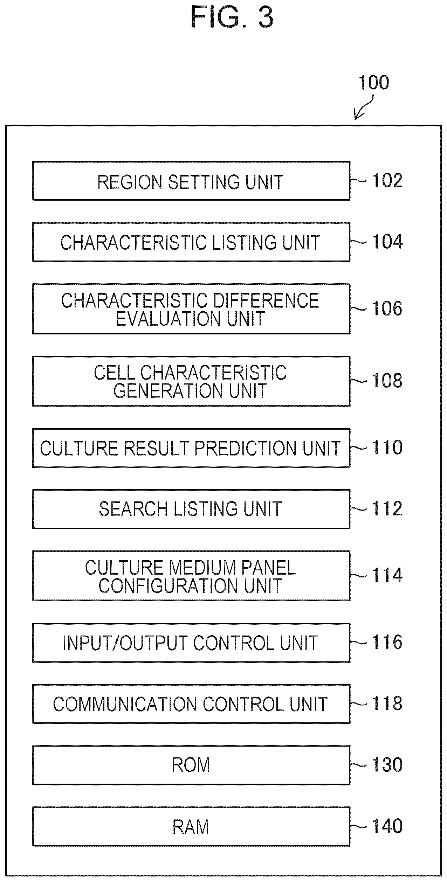

Fig. 3 is a block diagram illustrating a configuration of the processing unit 100 (processor). The processing unit 100 includes a region setting unit 102, a characteristic listing unit 104, a characteristic difference evaluation unit 106, a cell characteristic generation unit 108, a culture result prediction unit 110, a search listing unit 112, a culture medium panel configuration unit 114, an input/output control unit 116, a communication control unit 118, a ROM 130 (ROM: Read Only Memory), and a RAM 140 (RAM: Random Access Memory).

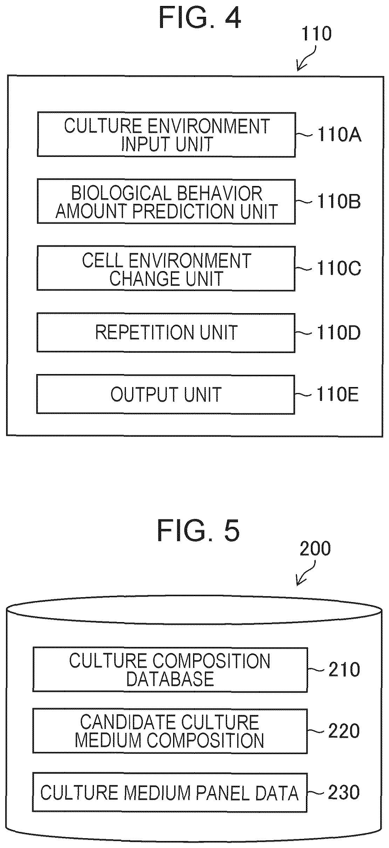

Fig. 4 is a block diagram illustrating a configuration of the culture result prediction unit 110 (processor). The culture result prediction unit 110 includes a culture environment input unit 110A, a biological behavior amount prediction unit 110B, a cell environment change unit 110C, a repetition unit 110D, and an output unit 110E.

The functions of the components of the processing unit 100 described above can be implemented using various processors. The various processors include, for example, a CPU (Central Processing Unit), which is a general-purpose processor that executes software (program) to implement various functions. The various processors described above also include a programmable logic device (PLD) that is a processor whose circuit configuration can be changed after manufacture, such as an FPGA (Field Programmable Gate Array). The various processors described above further include a dedicated electric circuit that is a processor having a circuit configuration designed specifically for executing specific processing, such as an ASIC (Application Specific Integrated Circuit).

The functions of the components may be implemented by a single processor or a combination of a plurality of processors. Alternatively, a plurality of functions may be implemented by a single processor. Examples of configuring a plurality of functions by a single processor include, first, a form in which, as typified by a computer such as a client or server computer, the single processor is configured by a combination of one or more CPUs and software and the processor is implemented as a plurality of functions. The examples include, second, a form in which, as typified by a system on chip (SoC) or the like, a processor is used in which the functions of the entire system are implemented by a single IC (Integrated Circuit) chip. As described above, the various functions are configured using one or more of the various processors described above as a hardware structure. More specifically, the hardware structure of the various processors is an electric circuit (circuitry) including a combination of circuit elements such as semiconductor elements.

When the processor or electric circuit described above is to execute software (program), the processor (computer) readable code of the software to be executed is stored in a non-transitory tangible recording medium, such as the ROM 130, and the processor refers to the software. The software to be stored in the non-transitory tangible recording medium includes a program for executing a method according to the present invention (a method for configuring a culture medium panel constituted by a plurality of culture media to optimize a cell culture result; hereinafter referred to as a "configuration method") (a configuration program for executing the method for configuring a culture medium panel constituted by a plurality of culture media to optimize a cell culture result; hereinafter referred to as a "configuration program"). The code may be recorded on a non-transitory tangible recording medium such as various magneto-optical recording devices or a semiconductor memory, instead of the ROM 130. In the processing using software, for example, the RAM 140 is used as a temporary storage area. For example, data stored in an EEPROM (Electronically Erasable and Programmable Read Only Memory) (not illustrated) may be referred to.

The term "non-transitory tangible recording medium", as described above, does not include a non-tangible recording medium such as a carrier signal or a propagation signal itself.

Processing using these components of the processing unit 100 will be described in detail below.

Configuration of Storage Unit

The storage unit 200 is constituted by a non-transitory tangible recording medium such as various magneto-optical recording media or a semiconductor memory, and an input/output control unit thereof, and stores information such as a culture medium composition, a cell characteristic, and a culture result. For example, as illustrated in Fig. 5, the storage unit 200 according to the present embodiment stores a culture medium composition database 210 (e.g., an experimental database), candidate culture medium compositions 220, and culture medium panel data 230 in association with each other.

Configuration of Display Unit and Operation Unit

The display unit 300 includes a monitor 310 (display device) and is capable of displaying input information, information stored in the storage unit 200, a result of processing performed by the processing unit 100, and so on. The operation unit 400 includes a keyboard 410 and a mouse 420 as an input device and/or a pointing device. The user can use these devices and a screen of the monitor 310 to perform an operation necessary to execute the configuration method and the configuration program according to the present invention.

Processing of Configuration Method and Configuration Program

The following describes processing of a configuration method and a configuration program according to the first embodiment with reference to Figs. 1 to 6. To simplify the description, some of the elements of the culture medium panel configuration system 10 are not illustrated, as necessary.

In the first embodiment, a description will be given of an example of configuring a culture medium panel for antibody-producing CHO cells, with the productivity to be optimized being the antibody yield thereof.

Setting of Region

The region setting unit 102 (processor) sets a region of the culture medium composition space (step S100; region setting step or region setting process). The term "region" corresponds to a search space for a culture medium composition to configure a culture medium panel. The region setting unit 102 may set a region on the basis of a culture medium composition recorded in the culture medium composition database 210 illustrated in Figs. 1 and 5 or may set a region on the basis of literature information including a catalog and the like. The region setting unit 102 may limit or expand composition components to be applied.

Listing of Cell Characteristics

The characteristic listing unit 104 (processor) lists a plurality of cell characteristics (step S110; characteristic listing step or characteristic listing process). The characteristic listing unit 104 may list characteristics on the basis of previous experiments or list characteristics on the basis of literature information such as an article when the characteristics are known cell characteristics. The characteristic listing unit 104 may further add an unknown cell characteristic (virtual cell characteristic) (for this expansion, see the "Listing and Expansion of Cell Characteristics" section). As used herein, the term "cell characteristic" can be biologically or cytologically defined in various ways. In the present invention, the term "cell characteristic" is defined as "a mechanism for determining the productivity of cells (such as the proliferative property or the antibody yield of cells, as described above) when a culture medium composition is given". Specifically, the term "cell characteristic" in the present embodiment refers to a mechanism for determining an antibody yield for a culture medium composition, and can be represented by, for example, a function, a machine learning model, or a cell mathematical model. Accordingly, for example, a coefficient of a function, a parameter of machine learning, or a parameter of a metabolic pathway model among cell mathematical models can be considered to be part of a cell characteristic in the present invention. A specific example of a cell characteristic will be described in conjunction with the culture result prediction unit 110 (processor) described below.

Prediction of Culture Result

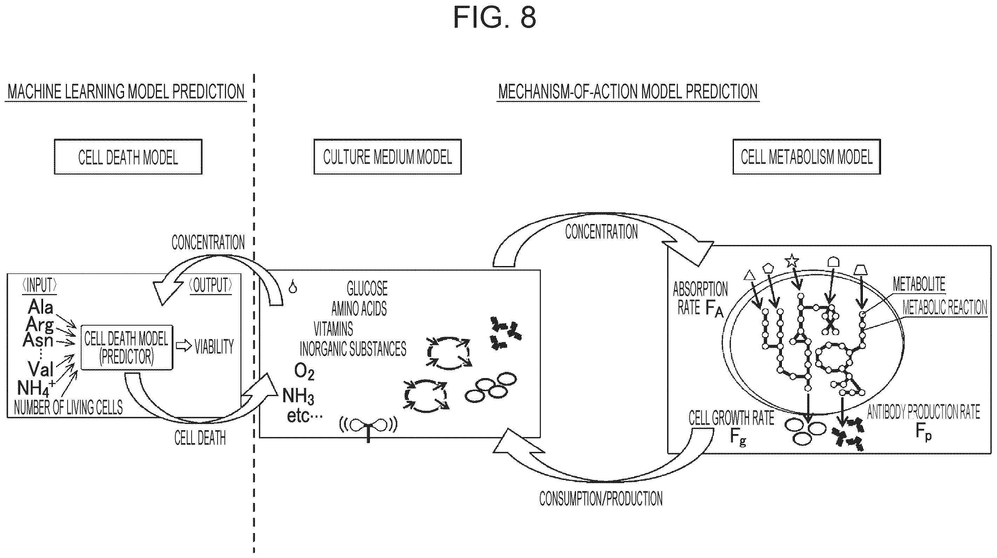

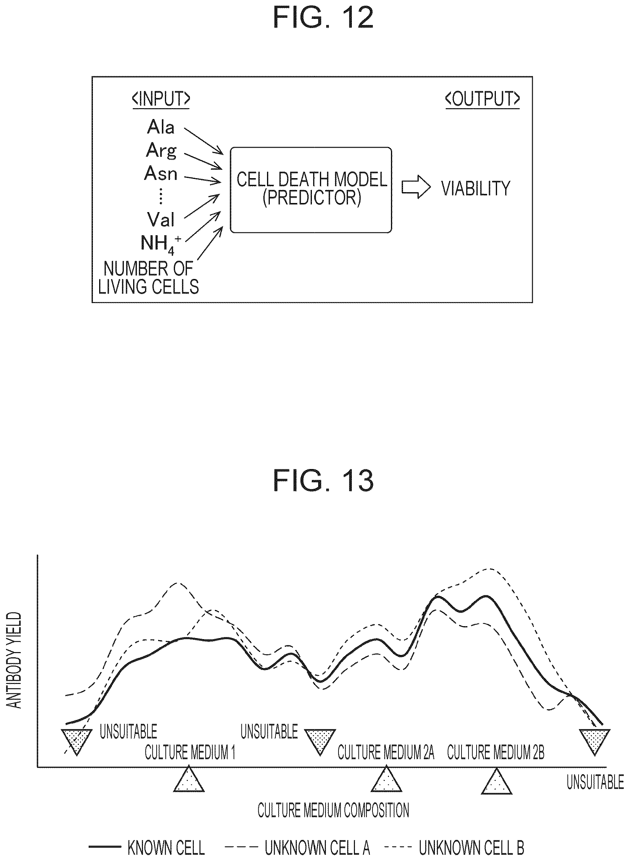

The characteristic listing unit 104 inputs a region S of the culture medium composition space (specifically, culture medium compositions s included in the region S $\in$ the region S) to the culture result prediction unit 110 on the basis of listed cell characteristics (cell characteristics C; see Fig. 1). Accordingly, the culture result prediction unit 110 predicts a culture result of the target cell in a culture medium having a given composition on the basis of a cell characteristic of the target cell (step S120; prediction step or prediction process). The culture result prediction unit 110 may be a function (specifically, a processor, a program, a memory, or the like that implements the functionality of the function), and may further be a predictor obtained by machine learning (see in Figs. 8 and 12, etc.), a simulator of a metabolic pathway model (cell simulation), or the like. In the first embodiment, such a cell simulation can be used to express at least one of a means for prediction in the prediction step or prediction process, a prediction function of an unknown cell, or a difference between cell characteristics.
The culture result prediction unit 110 may be constructed by measuring several points through an experiment and, for example, continuously and smoothly connecting the measured points, may be constructed by a machine learning model in which a culture medium composition is an explanatory variable and a culture result is an objective variable, or may be constructed as a metabolic pathway model. Accordingly, the cell characteristics C may be, for example, coefficients of a function serving as a predictor, a parameter of machine learning, a metabolic map in a metabolic pathway model, or the like.

Culture Result Prediction Method

An aspect of a culture result prediction method performed by the culture result prediction unit 110 described above will be described.

Method for Predicting Cell Culture Result

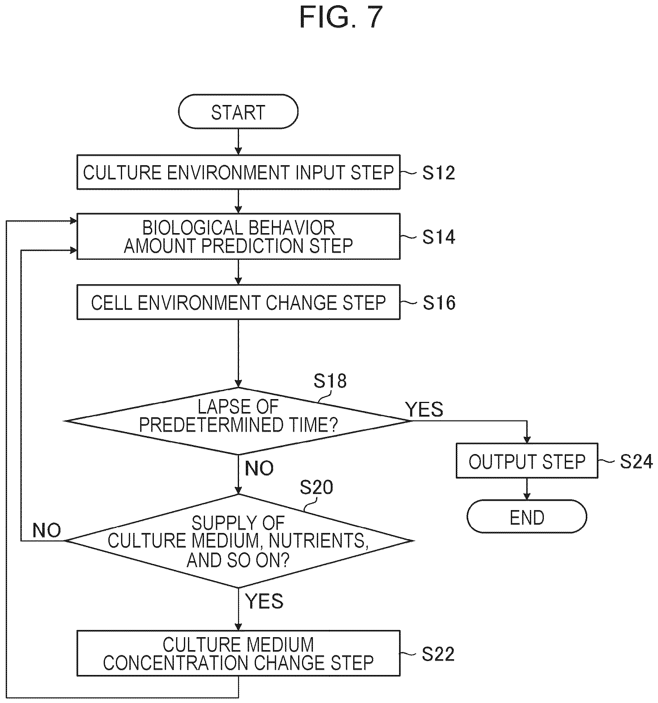

Fig. 7 is a flowchart illustrating a method for predicting a culture result of a target cell, which is performed by the culture result prediction unit 110 having the configuration described above. This method has a culture environment input step of inputting a culture environment constituted by a culture medium composition and culture conditions for culturing cells, a biological behavior amount prediction step of predicting a biological behavior amount on the basis of the culture environment input in the culture environment input step, and a cell environment change step of changing a cell environment on the basis of the biological behavior amount predicted in the biological behavior amount prediction step. The method further has a repetition step of repeating the biological behavior amount prediction step of predicting a biological behavior amount on the basis of the cell environment changed in the cell environment change step and the cell environment change step until a predetermined time that is a culture time elapses. The method further has an output step of outputting the cell environment changed in the cell environment change step after the predetermined time has elapsed.
The steps will be described hereinafter.

Culture Environment Input Step (Step S12)

The culture environment input unit 110A performs a culture environment input step (step S12). The culture environment input step is a step of inputting a culture environment constituted by a culture medium composition and culture conditions for culturing cells. The culture environment is input in accordance with the user's operation, and is processed by the culture environment input unit 110A.
Examples of the culture medium composition include culture medium components of a culture medium for culturing the cells and the amounts of the culture medium components. The culture conditions are setting conditions for optimizing a cell culture process. Examples of the culture conditions include conditions such as a culture method, the size and type of a culture vessel, addition of oxygen, supply of a culture medium and a nutrient source, addition of a pH adjuster and carbon dioxide, removal of a culture medium containing growth-inhibiting by-products, and harvest of a target product.

Examples of the culture method include whether to perform sterilization treatment, whether the culture medium is a liquid or a solid, and the culture temperature. The culture conditions are not limited to the conditions at the start of culture, and items during the culture period, such as the presence or absence of addition of oxygen and the amount of addition, the presence or absence of supply of a culture medium and a nutrient source and the amount of supply, addition of a pH adjuster and carbon dioxide, the presence or absence of removal of a culture medium containing growth-inhibiting by-products and the amount of supply, and the presence or absence of harvest of a target product and the amount of harvest can be set as culture conditions.

Biological Behavior Amount Prediction Step (Step S14)

The biological behavior amount prediction unit 110B performs a biological behavior amount prediction step (step S14). The biological behavior amount prediction step is a step of predicting a biological behavior amount on the basis of the culture environment input in the culture environment input step. The biological behavior amount includes a change in the total number of cells over time, changes in substances produced by the cells and the amounts of the substances over time, and changes in substances included in the culture environment and the amounts of the substances over time. The substances produced by the cells are antibodies and the like produced by the cells. The substances included in the culture environment are culture medium components, by-products, and the like.

Fig. 8 is a diagram illustrating an overview of a simulator for the biological behavior amount prediction step. As illustrated in Fig. 8, the biological behavior amount prediction step is performed by using a mechanism-of-action model (biological mechanism-of-action model) and a machine learning model. As the mechanism-of-action model, respective mathematical models for the inside and outside of a cell are used. For the inside of the cell, a cell metabolism model for calculating a cell growth rate $F_g$ and an antibody production rate $F_p$ by using an absorption rate $F_A$ at which the cell absorbs each component of the culture medium is used. For the outside of the cell, a culture medium model for calculating a change in the concentration of each component of the culture medium is used. For cell death, a model determined by using machine learning is used.

First, a cell metabolism model performed inside a cell will be described. In the cell metabolism model, the cell growth rate and the antibody production rate can be determined by using a cell culture simulation method that reproduces a bioprocess and the mechanism of a cell to be cultured. The cell culture simulation method can be performed by using a method including a modeling approach including flux balance analysis (FBA) or metabolic flux analysis (MFA) using a genome-scale metabolic model.

The "flux" or "metabolic flux" refers to the rate at which molecules pass through a target pathway or reaction. Among the factors that control flux are the rate of catalysis of enzymes in the pathway, the availability (durability) of substrate, the concentration of enzymes in the cell, and the proximity of enzymes in the pathway. The "metabolic flux analysis method" is a method for determining the amount of molecules that migrate from these factors. The "flux balance analysis" is an analysis method focusing on stoichiometry and metabolic flow rate, and, even when constants related to metabolism are not fully measurable, analyzes the behavior range of a target metabolic circuit and features thereof from the structure of a metabolic reaction on the basis of basic constraint conditions such as the law of conservation of mass.

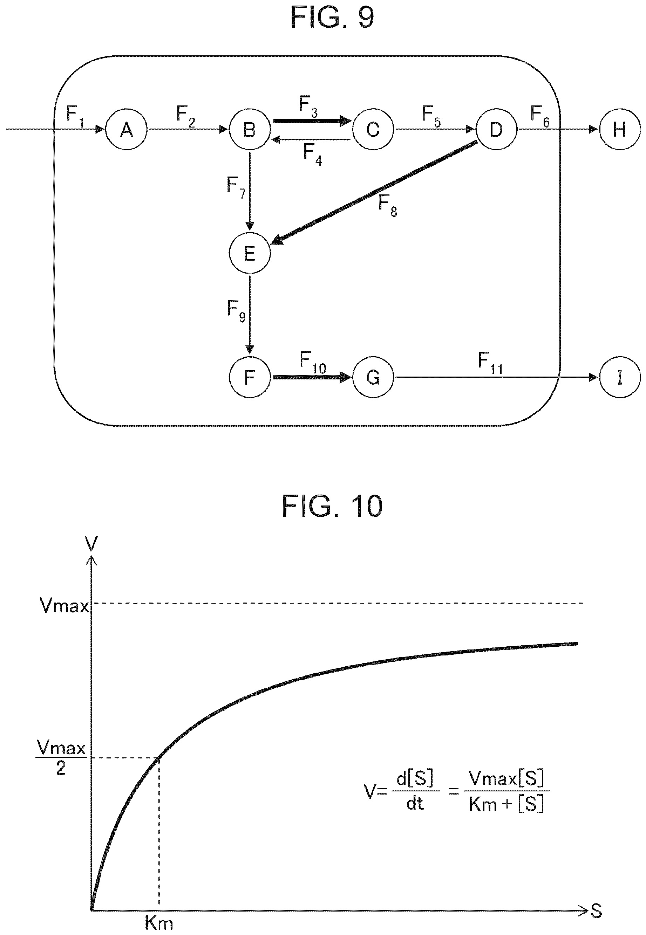

Next, intracellular metabolism will be described with reference to Fig. 9. Fig. 9 is a schematic diagram illustrating an example of cellular metabolic pathways. For a cell metabolism model, first, uptake constraint conditions, which are upper limits and lower limits for uptake of a substrate used for metabolism, nutrients, and the like in a culture medium, are acquired. In Fig. 9, substances generated by metabolism are represented by A, B, C, etc. $F_1$, $F_2$, $F_3$, etc. represent functions indicating the respective changes in the concentrations of the substances over time. For example, $F_1$ represents a flux at which the substance A is taken in, and $F_2$ represents a flux at which the substance B is made through metabolism.

In the example illustrated in Fig. 9, the conversion of the substance A into the substance B is performed in equal amounts in accordance with the law of conservation of mass. Likewise, the amount of the substance B is equal to the total amount of conversion into the substance C and the substance E. As described above, the amount of a substance to be converted is determined by the amounts of a substrate and a nutrient to be initially taken in by a cell, and so on. Another parameter that limits the conversion of a substance is the reaction rate. For example, as described above, the conversion of the substance A into the substance B is performed in equal amounts. However, in a certain unit time, the conversion into the substance B has an upper limit due to the limited rate of reaction, and not all the substance A may be converted into the substance B. This parameter for limiting the conversion of a substance is acquired as an uptake constraint condition, and the uptake constraint condition is acquired by using an expression obtained by mathematically modeling a mechanism by which a cell takes in a culture medium component, and is applied to calculation of a culture result.

As a mathematical model for determining the reaction rate, for example, the Michaelis-Menten equation can be used. Fig. 10 illustrates an overview of the Michaelis-Menten equation. The Michaelis-Menten equation is an equation related to a reaction rate V of an enzyme. When a substrate concentration S is low, the reaction rate V is proportional to the substrate concentration S. When the substrate concentration S is high, the reaction rate V converges to a maximum rate Vmax regardless of the substrate concentration S. Other mathematical models that can be used include Fick's law. Fick's law is an expression used to determine a flux that is the amount of substance passing through a unit area per unit time, and the flux is proportional to a diffusion coefficient D and a substrate concentration gradient on both sides of a membrane. In the present embodiment, the Michaelis-Menten equation or Fick's law is used as a constraint condition for taking in a culture medium. This makes it possible to perform a simulation without calculating a condition in which a reaction rate is higher than the reaction rate and the flux determined by the mathematical models but falls within the range of the amount of conversion determined by the law of conservation of mass. Accordingly, more accurate culture prediction can be performed.

The calculation by simulation may be performed by using, in parallel, a plurality of mathematical models that mimic the state of a cell. For example, a mathematical model for cell growth and a mathematical model for production of only bioproduction without cell growth can be arranged in parallel, and the ratio between the plurality of models can be changed in accordance with the culture state such as the culture medium concentration.

Next, a culture medium model performed outside a cell will be described. The culture medium model is a model for determining a change in the concentration of a culture medium surrounding a cell when metabolism occurs under the conditions of the cell metabolism model described above, and a cell signaling model represented by a differential equation can be used. Regarding the concentration change, the Runge-Kutta method can be used to solve an ordinary differential equation for the time t to determine the change in the concentration of each component.

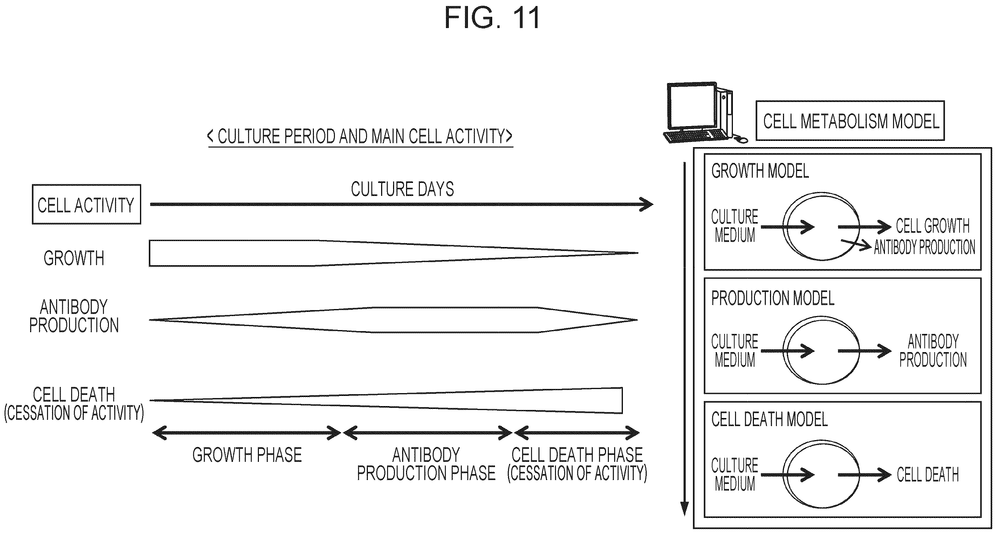

Existing cell metabolism models including a cell death model will now be described. Fig. 11 is a diagram illustrating an overview of existing cell metabolism models. The main cell culture activity during the culture period is divided into cell growth, antibody production by cells, and cell death (cessation of activity). As illustrated in Fig. 11, as the phases of these activities, the early stage of culture has a growth phase in which cells grow, and the middle stage of culture has an antibody production phase in which the cell growth rate decreases and the antibody production rate increases. Subsequently, the late stage of culture has a cell death phase in which cell death, which is the cessation of cellular activity, occurs. In an existing simulation for culture prediction, these three culture activities are performed by using a cell metabolism model, and calculation is performed by using a growth model for cell growth, a production model for antibody production, and a cell death model for cell death. However, the cell death model is implemented as a simple model with constants, and sufficient accuracy is not obtained. In the present embodiment, accordingly, as illustrated in Fig. 8, a trained model (an aspect of a predictor) obtained by machine learning is used as a cell death model to perform a simulation to calculate a prediction result of culture with high accuracy.

Fig. 12 is a schematic diagram illustrating creation of a cell death model by using machine learning. A random forest method can be used as a method for creating a trained model by machine learning. When a cell death model is constructed by machine learning, the number of living cells, the concentrations of 20 types of amino acids, and the concentration of ammonia are input as data to the input side. The cell viability is input as data to the output side. The data to be used is obtained by actually performing culture, and, for example, 80% of the measured data is used for learning and 20% thereof is used for testing. This makes it possible to create a cell death model (an aspect of a predictor).

The creation of a trained model by machine learning is not limited to that for a cell death model. For a data item that is difficult to model based on a mechanism, such as cell death, a trained model is created by machine learning to calculate a prediction result of culture with higher accuracy. Examples of such a data item include growth inhibition and suppression of antibody production. To create a growth inhibition model by machine learning, the amount of cell growth (growth ratio) can be used as data to be used for the output side. To create an antibody production suppression model, the amount of antibody production (production ratio) can be used as data to be used for the output side.

In the biological behavior amount prediction step according to the present embodiment, a trained model created by machine learning is used for cell death, thereby making it possible to measure a biological behavior amount with higher accuracy. As a result, a prediction result of culture can be calculated with high accuracy.

The biological behavior amount prediction step (step S14) can be performed, based on the culture conditions (concentrations of culture medium components) and the uptake constraint conditions acquired in the culture condition input step (step S12), by using a CHO cell simulator including these models (an apparatus or a system that simulates the biological behavior of CHO cells by using a mechanism-of-action model and a trained model created by machine learning for cell death).

Cell Environment Change Step (Step S16)

The cell environment change unit 110C performs a cell environment change step (step S16). The cell environment change step changes a cell environment on the basis of the biological behavior amount predicted in the biological behavior amount prediction step. The cell environment includes the total number of cells, substances produced by the cells, and the amounts of the substances.

Through the biological behavior amount prediction step, the change in the number of cells over time, the changes in substances produced by the cells and the amounts of the substances over time, and the changes in substances included in the culture environment and the amounts of the substances over time, described above, after a culture time t has elapsed are determined by calculation. In the cell environment change step, the changes in the number of cells and the components of the culture medium after the culture time t has elapsed are reflected in the cell environment.

Repetition Step

The repetition unit 110D controls implementation of a repetition step. The repetition step is a step of repeating the biological behavior amount prediction step (step S14) and the cell environment change step (step S16).

After the completion of the cell environment change step (step S16), the repetition unit 110D determines whether a predetermined culture time has elapsed (step S18). If the predetermined culture time (e.g., 14 days) for the calculation has elapsed (if YSE is determined), the calculation ends, and the process proceeds to an output step (step S24). If the predetermined culture time has not elapsed (if NO is determined), it is determined whether to supply a culture medium, nutrients, and the like (step S20). If no culture medium, nutrients, or the like is to be supplied (if NO is determined), the process returns to the biological behavior amount prediction step (step S14). If a culture medium, nutrients, and the like are to be supplied (if YES is determined), a culture medium concentration change step (step S22) is performed.

If the supply of a culture medium, nutrients, and the like after a predetermined time has elapsed is set as a culture condition in the culture environment input step (step S12), the culture medium concentration change step (step S22) is performed. The supply of a culture medium, nutrients, and the like also includes the supply of a pH adjuster and carbon dioxide in addition to the culture medium and the nutrients. The culture medium concentration change step adds the supplied culture medium and nutrients to the cell environment at the time point when the cell environment change step is completed to change the culture medium concentration.

The culture medium concentration change step can involve not only supplying a culture medium, nutrients, and the like but also changing the culture medium concentration by removal of a culture medium containing growth-inhibiting by-products and harvest of a target product. If the removal of a culture medium containing growth-inhibiting by-products and the harvest of a target product are set as culture conditions in the culture environment input step (step S12), the removed growth-inhibiting by-products and the harvested target product are removed from the cell environment at the time point when the cell environment change step is completed to change the culture medium concentration.

In the repetition step, an uptake constraint condition is acquired on the basis of the cell environment changed in the cell environment change step (step S16) or the culture medium concentration changed in the culture medium concentration change step (step S22), and the biological behavior amount prediction step (step S14) and the cell environment change step (step S16) are performed. Thereafter, the biological behavior amount prediction step (step S14) and the cell environment change step (step S16) are repeated until the predetermined culture time elapses (step S18).

After the predetermined culture period has elapsed (YES in step S18), the calculation ends. As a result, the number of cells during the culture period and the amount of generation of bioproduction, ammonia, or the like to be generated can be determined. Further, the biological behavior amounts at the respective time points when the repetition step is performed are plotted, thereby making it possible to determine the cell growth curve, the progress of antibody production, and so on.

Output Step (Step S24)

The output unit 110E performs the output step (step S24). The output step is a step of outputting the cell environment changed in the cell environment change step (step S16).

The output step can output, as results, the number of cells growing during the culture period and the amount of a bioproduction to be generated or the amount of generation of a bioproduction such as an antibody like ammonia. In addition, the cell growth curve, the progress of antibody production, and the like can be output. As the result to be output, the results described above after the lapse of the predetermined time may be output, or the results described above after the lapse of any period during the culture may be output.

Search and Listing of Culture Medium Compositions

Referring back to Figs. 1 to 6, the description will be continued. The search listing unit 112 (processor) uses the culture result prediction unit 110 (predictor) to extract culture medium composition candidates for which the cell culture results for various culture medium compositions s in the region S have local maximum values, and searches for and lists, for the respective cell characteristics included in the plurality of cell characteristics described above, culture medium compositions for which the cell culture results have local maximum values from the prediction of the culture results for the extracted culture medium composition candidates (step S130; search listing step or search listing process). The search listing unit 112 may search for a local maximum value as a search problem for function optimization. For example, if the gradient of the function serving as a predictor described above can be defined, the search listing unit 112 may search for a local maximum value by using Newton's method or its extended method, or may select a maximum value from a list of random values of the function serving as a predictor. The search listing unit 112 can store the obtained culture medium composition candidates (candidates R) in the storage unit 200 as the candidate culture medium compositions 220.

As is generally known, when a gradient can be defined or approximately calculated, the search listing unit 112 can calculate a convex point having a gradient of 0 in a set region as a "local maximum value". Even a non-differentiable gradient can be approximately calculated from the values of two neighboring points by determining the values of adjacent points and taking a difference. Alternatively, the search listing unit 112 may randomly calculate values, cut out a partial region therefrom as appropriate, and determine a local maximum value. Other optimization methods, such as the Nelder-Mead method, which does not use any derivatives, may also be used. The search listing unit 112 may apply any of various other methods for optimizing a linear or nonlinear function to determine a local maximum value.

Configuration of Culture Medium Panel

The culture medium panel configuration unit 114 (processor) configures a culture medium panel from the culture medium composition candidates R obtained in step S130 (step S140; configuration step or configuration process). The details of the configuration of a culture medium panel will be described below.

Presentation of Culture Medium Panel

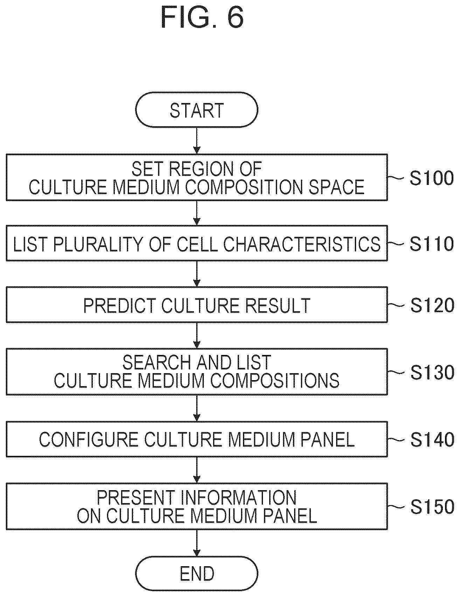

The input/output control unit 116 (processor) presents information on the obtained culture medium panel (step S150 in Fig. 6; information presentation step or information presentation process). The input/output control unit 116 may store the information on the culture medium panel in the storage unit 200 as the culture medium panel data 230 (see Fig. 5), or may provide the information on the culture medium panel to the external server 500, the external database, or the like via the communication control unit 118 and the network NW.

Listing and Expansion of Cell Characteristics

The characteristic listing unit 104 may list cell characteristics by generating and adding an unknown cell characteristic (virtual cell characteristic). That is, a cell characteristic can be generated by the cell characteristic generation unit 108 (characteristic generator) on the basis of a difference constraint L (constraint condition on differences) from known cell characteristics (see Fig. 1). For example, when the cell characteristics C are defined as coefficients of a function F (an aspect of the culture result prediction unit 110), the difference constraint L may be the range of variation of the coefficients; when the cell characteristics C are defined as parameters of machine learning, the difference constraint L may be the range of variation of the parameters; or the difference constraint L may be an inter-model difference such as the Hamming distance between metabolic pathway models in a network matrix representation.

These can also be measured between known cell characteristics, and thus a characteristic difference between an unknown cell and a known cell can be set with reference to the measured difference. That is, the difference constraint L may be based on literature information or the like. Alternatively, the characteristic difference between known cell characteristics may be measured (evaluation step or evaluation process of evaluating a characteristic difference between cells), and the cell characteristic generation unit 108 (characteristic generator) may set the difference constraint L with reference to the measured characteristic difference (e.g., to an equivalent value or a value multiplied by a constant). The cell characteristic generation unit 108 can generate, based on the set difference constraint L, a characteristic (virtual cell characteristic) of an unknown cell whose difference from the cell characteristics of a known cell group satisfies the difference constraint L (constraint condition) while controlling the characteristic of the unknown cell (generation step or generation process). The characteristic listing unit 104 can list a plurality of cell characteristics in the characteristic listing step or the characteristic listing process such that one or more of the unknown cell characteristics (virtual cell characteristics) generated in the way described above are included in the plurality of cell characteristics.

Details of Configuration of Culture Medium Panel

The configuration of a culture medium panel (i.e., in Fig. 1, the configuration of a culture medium panel P from the candidates R) will be described with reference to Fig. 13. In Fig. 13, the horizontal axis represents the culture medium composition, and the vertical axis represents the antibody yield. A solid line indicates the antibody yields of a known cell for various culture medium compositions, and is a result of prediction of a culture result by a predictor (the culture result prediction unit 110) such as a function on the basis of the known cell characteristics. While a single line is illustrated, a plurality of lines may be illustrated. In contrast, broken lines indicate the antibody yields of unknown cells, and are also results of prediction of culture results by the predictor on the basis of the unknown cell characteristics.

The culture medium panel configuration unit 114 (processor) selects a culture medium to be added to the culture medium panel from the shapes of the solid line and the broken lines. A local maximum value or the vicinity thereof is a strong candidate, and in particular, a point at which local maximum values are close to each other for a plurality of cells is more preferable. For example, points indicated by upward triangles △ (culture medium 1, culture medium 2A, and culture medium 2B) are examples of suitable points, and points indicated by downward triangular triangles ▽ (unsuitable) are examples of unsuitable points.

Furthermore, if various culture media are selected, an unknown cell having slightly deviating characteristics may also be supported, which is considered to be redundant. For example, if two of the three candidates described above are to be selected, the culture medium 1 and the culture medium 2B are desirable because it is preferable to select only the culture medium 2B from among the culture media 2A and 2B having similar compositions. Since the culture medium 1 is expected to be particularly preferable for unknown cell A while having a slightly lower antibody yield than the culture medium 2A and the culture medium 2B for the known cell, the culture medium panel configuration unit 114 desirably selects a culture medium from among such candidates.

A variety of culture media can be abstractly referred to as a "way of selection that widens the distribution of culture medium composition candidates in the culture medium composition space". The culture medium panel configuration unit 114 may select, as the "way of selection that widens the distribution of culture medium composition candidates in the culture medium composition space", for example, candidate culture media between which the correlation decreases in the configuration of a culture medium panel, select culture media for which the distance between candidate culture media increases, or select candidate culture media for which diversity definable from the information gain increases. When a culture medium is added to the culture medium panel, information on a cell characteristic corresponding to the added culture medium (a culture result in the culture medium; specifically, for example, the antibody yield) can be obtained, and thus the addition of a culture medium to the culture medium panel can be considered to be virtual "observation" related to a cell characteristic. In this case, when it is expected that the uncertainty of a cell characteristic of an unknown cell will greatly decrease by adding a culture medium, the culture medium is a "culture medium with a large information gain". When it is expected that the uncertainty will not greatly decrease even by adding a culture medium, the culture medium is a "culture medium with a small information gain". A culture medium with a large information gain is a culture medium having high diversity. It is preferable that the culture medium panel configuration unit 114 preferentially add such a culture medium to the culture medium panel. As described above, the use of an information gain makes it possible to ensure the diversity of a culture medium in consideration of the uncertainty of a function corresponding to a cell characteristic.

The culture medium panel configuration unit 114 can calculate the correlation or distance between culture medium compositions by directly comparing the culture medium compositions.

In the example in Fig. 13, although the cell characteristics are considered to be relatively close to each other, since a new composition particularly suitable for a new cell may be present, as in the culture medium 1, it can be said that selecting culture media having various compositions is particularly effective. That is, for example, when an actually obtained unknown cell X has a characteristic close to that of A, a satisfactory culture result can be expected by using the culture medium 1, and when the actually obtained unknown cell X has a characteristic close to that of B, a satisfactory culture result can be expected by using the culture medium 2B. As described above, according to the present invention, configuring a culture medium panel (set) constituted by not necessarily a single culture medium but rather a plurality of culture media can increase the suitability for culture media having various characteristics.

The culture medium panel configuration unit 114 may weight the culture medium composition candidates on the basis of the frequency of appearance or the probability of a cell characteristic and configure a culture medium panel on the basis of the result of the weighting. For example, in the example in Fig. 13, assuming that a cell characteristic similar to that of unknown cell B has a high frequency of appearance, the culture medium panel configuration unit 114 may select both the culture media 2A and 2B when a culture medium can further be added to the culture medium panel (when there is room for a number of culture media). The culture medium panel configuration unit 114 may perform any weighting on the basis of the frequency of appearance or the probability, or may determine that, for an unknown characteristic, the larger the difference from a known characteristic, the lower the frequency of appearance or the probability (the smaller the weight), in accordance with the difference.

An example of configuring a culture medium panel on the basis of the frequency of appearance of a cell characteristic will be described. For example, as a result of applying a certain culture medium panel to an existing cell ten times, if there are seven records of being suitable for cell characteristic A and three records of being suitable for cell characteristic B, the frequency of appearance (or the probability) can be represented as A = 0.7 and B = 0.3. Thus, a larger number of culture medium compositions having a local maximum value of a culture result for the cell characteristic A may be employed in the culture medium panel.

The culture medium panel configuration unit 114 may use, as the frequency of appearance of a cell characteristic, for example, information actually given by listening to a customer (a person who is provided with a culture medium panel configured according to the present invention and cultures cells) or the like, information determined on the basis of a performance record (e.g., information indicating that the frequency of appearance for cells closer to previous customer cells is higher), or information determined by simulation (e.g., a characteristic generated more frequently in a step of refining a virtual cell characteristic is regarded as having a "high frequency of appearance").

Extension of Configuration of Culture Medium Panel: Utilization of Cell Lineage

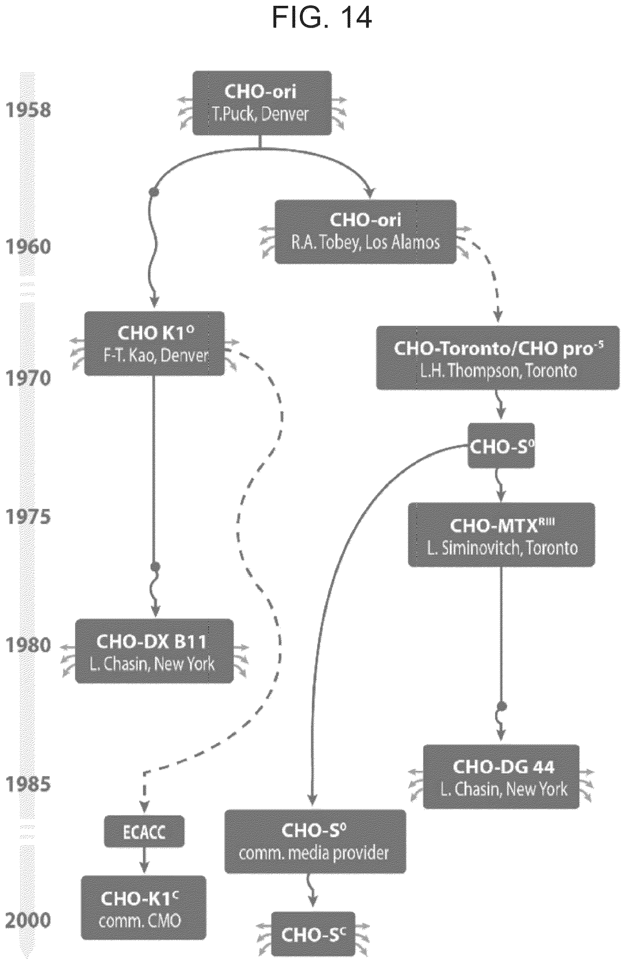

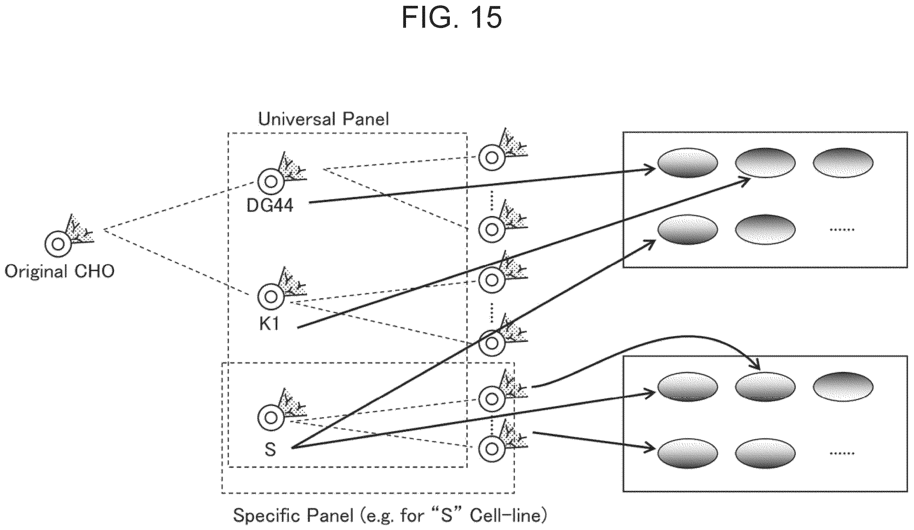

Next, an additional aspect of configuring a culture medium panel will be described with reference to Figs. 14 and 15. For example, for CHO cells, it is known that various CHO cell lines are not "equally spaced" from each other, and a relationship like a phylogenetic tree can be schematically represented by a derivation relationship or the like. Specifically, as illustrated in Fig. 14, CHO cells include an original CHO cell (CHO-ori) and a plurality of cell lines having different characteristics from the original CHO cell (CHO-ori), such as "CHO-K1", "CHO-S", and "CHO-DG44".

The phylogenetic tree in Fig. 14 is described in "Wurm, F.M.; Wurm, M.J. Cloning of CHO Cells, Productivity and Genetic Stability A Discussion. Processes 2017, 5, 20.", searched on March 1, 2022 on the Internet (https://www. researchgate.net/publication/316314849_Cloning_of_CHO_Cells_Productivity_ and_Genetic_Stability-A_Discussion/-link/59410f0045851566e1beae32/download).

The above article is cited in compliance with http://creativecommons.org/licenses/by/4.0/.

The relationship like a phylogenetic tree described above is not limited to that of CHO cells, and a phylogenetic tree is also present for HEK cells. Such a relationship may also occur when iPS cells are cultured for a long time. The term "cells", as used in the present invention, refers to host cells having certain genes and antibody-producing cells (also referred to as "cell lines") obtained by inserting antibody-producing genes into the host cells, which are generally established in the way described above. Such lineages and cell lines may be defined by the genes or may be defined with reference to commonly known lineages (in the case of CHO cells, "CHO-K1", "CHO-S", "CHO-DG44", etc.). The term "cell line" is defined in various ways, and can be defined as, for example, "cells isolated from a living body or cells that are obtained by modifying genes or the like in some way and are capable of long-term stable proliferation and culture while maintaining certain properties".

The culture medium panel configuration unit 114 may use this relationship to segment the culture medium panel into subsets (small subsets of the culture medium panel may be constructed within the culture medium panel; Fig. 15). That is, for example, in consideration of supporting all possible cells (in the example in Fig. 15, all DG44/K1/S lineages), the size of the culture medium panel may be increased, or the culture medium compositions to be set may be coarse (Universal Panel). The "Universal Panel" is an example of a "subset independent of a specific cell lineage". Accordingly, if the lineage of an unknown cell can be known, at least one or more known cell lineages are associated with any of the subsets (e.g., limiting any of the subsets to a known cell lineage S), thereby making it possible to configure a culture medium panel having a more dense association (Specific Panel) while reducing the size of the panel. This "Specific Panel" is an example of a "subset dependent on a specific cell lineage".

Then, when an unknown cell is actually given, the culture medium panel configuration unit 114 may selectively use either the Universal Panel or the Specific Panel as a culture medium panel to be applied on the basis of the prior knowledge of the cell characteristics of the unknown cell (such that, for example, the corresponding Specific Panel is provided if only the lineage is known or the Universal Panel is provided if the lineage and the characteristics are unknown).

If the lineage of an unknown cell cannot be known, the culture medium panel configuration unit 114 may segment the culture medium panel into subsets such that a subset independent of a specific cell lineage (the Universal Panel described above) is included in the subsets constituting the culture medium panel. For example, in a case where three culture media of a culture medium panel are to be determined, if there are three cell lineages, the culture medium panel configuration unit 114 employs, for a Universal Panel", a culture medium suitable for a representative cell of each lineage. Accordingly, it is expected that suitability will not be significantly impaired for cells of any cell lineage. On the other hand, when a culture medium panel for, for example, the cell lineage S (one lineage of CHO cells; see Fig. 14) is to be constituted by a subset (Specific Panel) dependent on a specific cell lineage, the culture medium panel configuration unit 114 employs a culture medium suitable for a representative cell S of the lineage S and cells S1 and S2 derived from the representative cell S. Accordingly, the configuration of such a culture medium may be employed only when the cell lineage is known to be the lineage S, although the suitability may be low for an unexpected cell lineage (e.g., the cell lineage K or the like). This makes it possible to expect a better culture result than a culture medium panel constituted by only culture media suitable for the cell lineage S, for example, for cells having characteristics close to those of the cells S1 and S2.

Application of Submodular Optimization Method

The culture medium panel configuration unit 114 may further apply a submodular optimization method to configure a culture medium panel. "Submodular optimization" is an optimization method using the submodularity of a set function. "Submodularity" is a concept corresponding to convexity or concavity (in a continuous function) defined for a set, and is known to be applicable to combinatorial optimization.

Specifically, a maximum antibody yield $P(M)$ for a cell with the cell characteristics C and a culture medium panel M is defined by Expression (1) below.

$$P(M) = \max\{F\_C(m \in M)\} \quad (1)$$

While the culture medium panel M can be any element in the culture medium composition space S, the culture medium composition space S may have, as an element, a local maximum value predicted by a predictor F (the culture result prediction unit 110) or the vicinity thereof, as described above. At this time, it is an object of the present invention to maximize the maximum antibody yield P(M) when the unknown cell characteristics C are given, and Expression (2) below is satisfied for any culture medium k.

$$P(M \cup \{k\}) = \max\{P(M), F\_C(k)\} \quad (2)$$

Accordingly, when another culture medium panel $N \subset M$ is considered, Expressions (3) and (4) below are satisfied.

$$P(N \cup \{k\}) - P(N) = \max\{0, F\_C(k) - P(N)\} \quad (3)$$

$$P(M \cup \{k\}) - P(M) = \max\{0, F\_C(k) - P(M)\} \quad (4)$$

In addition, since $P(M) \geq P(N)$ is apparent, Expression (5) below is satisfied.

$$P(N \cup \{k\}) - P(N) \geq P(M \cup \{k\}) - P(M) \quad (5)$$

Accordingly, the maximum antibody yield P(M) is a submodular function. When the size of a culture medium panel is represented by t, the problem of configuring an optimum culture medium panel for a (single) given cell characteristic can be expressed as Expression (6) below.

$$\operatorname{argmax}\_M\{P(M)\}, |M| \leq t \quad (6)$$

This problem is an NP-hard problem, and an approximate solution to the problem is known (for example, Nemhauser, George L., Laurence A. Wolsey, and Marshall L. Fisher. "An analysis of approximations for maximizing submodular set functions - I." Mathematical programming 14.1 (1978): 265-294., searched on March 1, 2022 on the Internet (https://www.cs.toronto.edu/~eidan/papers/submod-max.pdf)).

The present invention particularly deals with a case where a plurality of cell characteristics may be present. This can also be regarded as a problem of improving, as much as possible, the "worst result" of the configured culture medium panel for all the cell characteristics to be considered. At this time, when a set obtained by listing cell characteristics C to be considered by using the method of the present invention described above is represented by C', the submodular optimization method may be extended and defined as a problem of maximizing the minimum value among a plurality of submodular functions, as in Expression (7) below.

$$\operatorname{argmax}\_M\{\min\_(C \in C')\{P(M, C) = \max\{F\_C(m \in M)\}\}\}, |M| \leq t \quad (7)$$

An approximate solution to this problem is also known as an extended submodular function (for example, Krause, Andreas, et al. "Robust Submodular Observation Selection." Journal of Machine Learning Research 9.12 (2008), searched on March 1, 2022 on the Internet (https://jmlr.csail.mit.edu/papers/volume9/krause08b/krause08b.pdf)). Thus, such a solution may be applied.

That is, an aspect of the present invention is applied to a submodular optimization method to propose a solution to a problem by assigning specific components such as cells, cell characteristics, and culture medium compositions and further introducing domain-specific elements.

Application of Simulation Model

As described above, a cell simulator or a similar function thereof may be applied to the function corresponding to the predictor F (an aspect of the culture result prediction unit 110), the cell characteristics C, and so on.

EXAMPLE

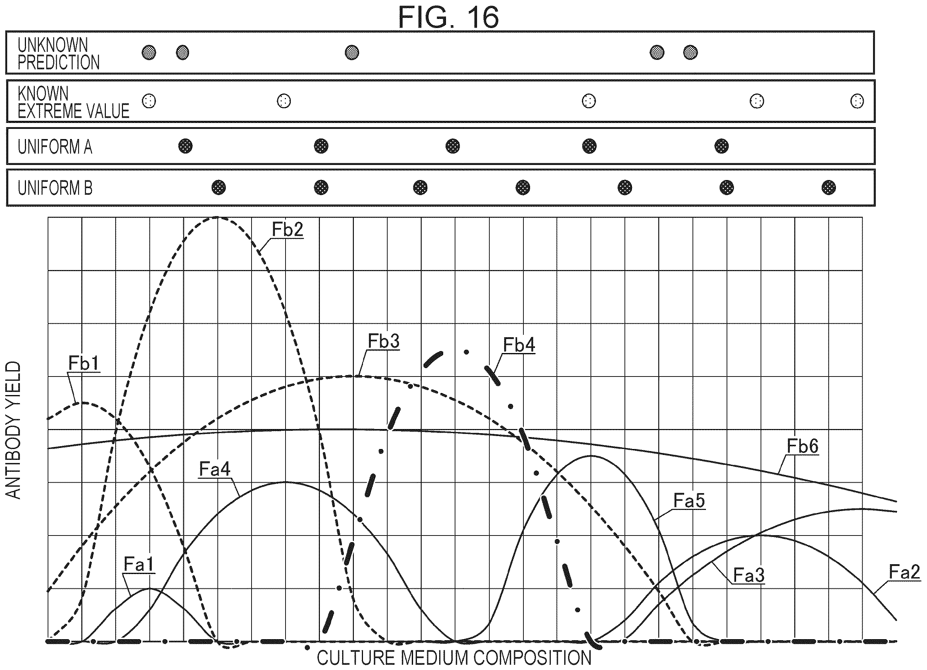

A search effect obtained by simulation is presented. A cell simulator (the predictor F; the culture result prediction unit 110) was constructed by using the method described in the "Culture Result Prediction Method" section, and an approximate function shape was observed to reproduce unknown cells and known cells. The general tendency is schematically illustrated in Fig. 16. A solid line indicates the relationship between the culture medium composition and the antibody yield for a known cell (so-called the predictor F). A broken line corresponds to a possible unknown cell, and a chain line corresponds to an impossible unknown cell. (It is assumed that the unknown cells have a characteristic in which a culture medium composition having an extreme value is close to that of any of the known cells)

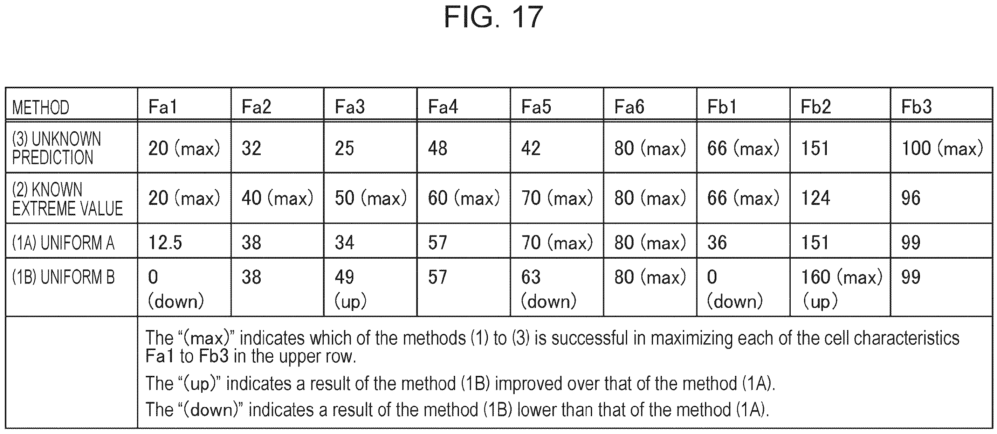

In contrast, Fig. 17 illustrates results of comparison among the following four methods.

(Method 1A) A method for uniformly arranging culture medium compositions on the assumption of a conventional method ("Uniform A" in Fig. 17)
(Method 1B) A method for uniformly arranging culture medium compositions on the assumption of the conventional method ("Uniform B" in Fig. 17), where the size of a culture medium panel is larger than that in any other method (the number of culture media included in the culture medium panel is larger than that in any other method by two)
(Method 2) A method for sequentially arranging culture medium compositions at local maximum values of known characteristics in such a manner as to be spaced a certain distance apart from each other ("Known Extreme Value" in Fig. 17; an aspect of the present invention)
(Method 3) A method for arranging culture medium compositions in such a manner as to not only make the culture medium compositions correspond to local maximum values of known characteristics but also take into account a characteristic of an unknown cell and not only simply arrange the compositions to be spaced apart from each other but also densely arranging the compositions in a region having a high variation in value ("Unknown Prediction" in Fig. 17; an aspect of the present invention)

As a result, at least one of Methods 2 and 3 according to aspects of the present invention provided the best result, and the best result was not provided only by Methods 1. In particular, Method 2 was always the best for known cells, and Method 3 was always the best for possible unknown cells. Specifically, the following results were obtained.

<Method 1A: Uniform> When culture medium compositions are uniformly arranged, the culture medium compositions may accidentally correspond to impossible unknown cells, but, in general, a culture medium different from a culture medium composition for which the result (the antibody yield; an example of a culture result) for known cells has a local maximum value may be "wastefully" selected, and it is difficult to uniformly arrange the culture medium compositions in a high-dimensional culture medium.

<Method 1B: Uniform> Although results for some cell characteristics (such as Fb2) are improved due to a shift in the setting range or interval of the culture medium compositions, the result for Fa5 is worse than that of Method 1A, and the results for Fa1 and Fb1 are hardly suitable.

<Method 2: Known Extreme Value> (one embodiment of the present invention) It was found that sequentially arranging culture medium compositions at local maximum values for known characteristics in such a manner as to be spaced a certain distance apart from each other made it possible to address a possible unknown characteristic to a certain extent.

<Method 3: Known Extreme Value> (one embodiment of the present invention) It was found that arranging culture medium compositions in such a manner as to not only make the culture medium compositions correspond to local maximum values for known characteristics but also take into account a characteristic of an unknown cell and not only simply arrange the compositions to be spaced apart from each other but also densely arranging the compositions in a region having a high variation in value made it possible to satisfactorily address even unknown characteristics.

As described above, it was confirmed that an embodiment of the present invention in which local maximum values of cell culture results are taken into account enabled configuration of a culture medium panel optimum for cells having a small size and various characteristics. Effect of First Embodiment

As described above, the culture medium panel configuration system 10 (configuration method, configuration apparatus, and configuration program) according to the first embodiment enables configuration of a culture medium panel having high suitability for cells having various characteristics, and further enables configuration of a culture medium panel also having high suitability for cells having a small size and unknown characteristics.

While an embodiment and other examples of the present invention have been described, the present invention is not limited to the aspects described above and may be modified in various ways.

Reference Signs List

| | |
|---|---|
| 1 | culture medium |
| 2A | culture medium |
| 2B | culture medium |
| 10 | culture medium panel configuration system |
| 100 | processing unit |
| 102 | region setting unit |
| 104 | characteristic listing unit |
| 106 | characteristic difference evaluation unit |
| 108 | cell characteristic generation unit |
| 110 | culture result prediction unit |
| 110A | culture environment input unit |
| 110B | biological behavior amount prediction unit |
| 110C | cell environment change unit |
| 110D | repetition unit |
| 110E | output unit |
| 112 | search listing unit |
| 114 | culture medium panel configuration unit |
| 116 | input/output control unit |
| 118 | communication control unit |
| 200 | storage unit |
| 210 | culture medium composition database |
| 220 | candidate culture medium composition |
| 230 | culture medium panel data |
| 300 | display unit |
| 310 | monitor |
| 400 | operation unit |
| 410 | keyboard |
| 420 | mouse |
| 500 | external server |
| 510 | external database |
| S12 to S24 | step of culture result prediction method |
| S100 to S150 | step of culture medium panel configuration method |

**Claims**

1. A configuration method for configuring a culture medium panel constituted by a plurality of culture media to optimize a cell culture result, the configuration method comprising, by a processor:

    a region setting step of setting a region of a culture medium composition space;
    a characteristic listing step of listing a plurality of cell characteristics;
    a prediction step of making a prediction of a culture result of a target cell for a given culture medium composition, based on a cell characteristic of the target cell;
    a search listing step of searching for and listing, for respective cell characteristics included in the plurality of cell characteristics, culture medium compositions for which cell culture results have local maximum values; and
    a configuration step of configuring the culture medium panel from culture medium composition candidates corresponding to the local maximum values, wherein
    in the search listing step, the processor
    extracts a culture medium composition candidate from the region of the culture medium composition space,
    predicts, for the extracted culture medium composition candidate, a culture result of a cell having a cell characteristic included in the plurality of cell characteristics in the prediction step, and
    searches for and determines, from the predicted culture result, culture medium composition candidates that provide the local maximum values.

2. The configuration method according to claim 1, wherein

in the configuration step, the processor configures the culture medium panel such that a distribution of the culture medium composition candidates in the culture medium composition space is widened.

3. The configuration method according to claim 2, wherein
in the configuration step, the processor configures the culture medium panel such that a correlation between the culture medium composition candidates in the culture medium composition space decreases, the culture medium panel such that a distance between the culture medium composition candidates in the culture medium composition space increases, or the culture medium panel such that diversity defined by an information gain of the culture medium composition candidates in the culture medium composition space increases.

4. The configuration method according to any one of claims 1 to 3, wherein
in the configuration step, the processor weights the culture medium composition candidates, based on a frequency of appearance of a cell characteristic, and configures the culture medium panel, based on a result of weighting of the culture medium composition candidates.

5. The configuration method according to any one of claims 1 to 4, comprising, by the processor:

an evaluation step of evaluating a characteristic difference between cells; and
a generation step of generating, based on the evaluated characteristic difference, virtual cell characteristics whose differences from a cell characteristic of a known cell group satisfy a constraint condition, while controlling the virtual cell characteristics, wherein
the plurality of cell characteristics to be listed in the characteristic listing step includes one or more of the generated virtual cell characteristics.

6. The configuration method according to claim 5, wherein
in the generation step, the processor sets the constraint condition, based on a characteristic difference between cells in the known cell group.

7. The configuration method according to any one of claims 1 to 6, wherein
the processor segments the culture medium panel into subsets and associates at least one or more known cell lineages with any one of the subsets.

8. The configuration method according to claim 7, wherein
the processor segments the culture medium panel into the subsets such that the subsets include a subset independent of a specific cell lineage.

9. The configuration method according to any one of claims 1 to 8, wherein
in the configuration step, the processor configures the culture medium panel by using a submodular optimization method.

10. The configuration method according to any one of claims 1 to 9, wherein
the processor uses a cell simulation including a metabolic pathway model to express at least one of a means for the prediction in the prediction step, a prediction function of an unknown cell, or a difference between cell characteristics.

11. The configuration method according to any one of claims 1 to 10, wherein
in the prediction step, the processor makes a prediction of the culture result by:

predicting a biological behavior amount of the target cell, based on the given culture medium composition and a culture condition;
changing a cell environment of the target cell, based on a prediction result of the biological behavior amount; and
repeating a prediction of the biological behavior amount and a change of the cell environment, based on the changed cell environment.

12. The configuration method according to claim 11, wherein

the processor
performs the prediction of, as the biological behavior amount, a change in a total number of cells over time, a change in a substance produced by the target cell and an amount of the substance over time, and a change in a substance included in a culture environment of the target cell and an amount of the substance over time, and performs the change of, as the cell environment, a total number of cells of the target cell, the substance produced by the target cell, and an amount of the substance.

13. The configuration method according to any one of claims 1 to 12, wherein the target cell is any one of a CHO cell, an HEK cell, or an iPS cell.

14. The configuration method according to any one of claims 1 to 13, wherein the culture result is one or more of an antibody yield or a virus yield by the target cell, a proliferative property of the target cell, and a success rate of inducing differentiation of the target cell into a specific tissue.

15. A configuration apparatus for configuring a culture medium panel constituted by a plurality of culture media to optimize a cell culture result, the configuration apparatus comprising a processor configured to perform:

 a setting process to set a region of a culture medium composition space;
 a listing process to list a plurality of cell characteristics;
 a prediction process to make a prediction of a culture result of a target cell for a given culture medium composition, based on a cell characteristic of the target cell;
 a search listing process to search for and list, for respective cell characteristics included in the plurality of cell characteristics, culture medium compositions for which cell culture results have local maximum values; and
 a configuration process to configure the culture medium panel from culture medium composition candidates corresponding to the local maximum values, wherein
 in the search listing process, the processor is configured to:

  extract a culture medium composition candidate from the region of the culture medium composition space, predict, for the extracted culture medium composition candidate, a culture result of a cell having a cell characteristic included in the plurality of cell characteristics in the prediction process, and
  search for and determine, from the predicted culture result, culture medium composition candidates that provide the local maximum values.

16. A configuration program for causing a processor to execute a configuration method for configuring a culture medium panel constituted by a plurality of culture media to optimize a cell culture result, the configuration method comprising:

 a region setting step of setting a region of a culture medium composition space;
 a characteristic listing step of listing a plurality of cell characteristics;
 a prediction step of making a prediction of a culture result of a target cell for a given culture medium composition, based on a cell characteristic of the target cell;
 a search listing step of searching for and listing, for respective cell characteristics included in the plurality of cell characteristics, culture medium compositions for which cell culture results have local maximum values; and
 a configuration step of configuring the culture medium panel from culture medium composition candidates corresponding to the local maximum values, wherein
 the search listing step
 extracts a culture medium composition candidate from the region of the culture medium composition space, predicts, for the extracted culture medium composition candidate, a culture result of a cell having a cell characteristic included in the plurality of cell characteristics in the prediction step, and
 searches for and determines, from the predicted culture result, culture medium composition candidates that provide the local maximum values.

17. A non-transitory computer-readable recording medium storing the program according to claim 16.

# FIG. 1

# FIG. 2

# FIG. 3

100

| | |
|---|---|
| REGION SETTING UNIT | ~102 |
| CHARACTERISTIC LISTING UNIT | ~104 |
| CHARACTERISTIC DIFFERENCE EVALUATION UNIT | ~106 |
| CELL CHARACTERISTIC GENERATION UNIT | ~108 |
| CULTURE RESULT PREDICTION UNIT | ~110 |
| SEARCH LISTING UNIT | ~112 |
| CULTURE MEDIUM PANEL CONFIGURATION UNIT | ~114 |
| INPUT/OUTPUT CONTROL UNIT | ~116 |
| COMMUNICATION CONTROL UNIT | ~118 |
| ROM | ~130 |
| RAM | ~140 |

# FIG. 4

110

| | |
|---|---|
| CULTURE ENVIRONMENT INPUT UNIT | 110A |
| BIOLOGICAL BEHAVIOR AMOUNT PREDICTION UNIT | 110B |
| CELL ENVIRONMENT CHANGE UNIT | 110C |
| REPETITION UNIT | 110D |
| OUTPUT UNIT | 110E |

# FIG. 5

200

| | |
|---|---|
| CULTURE COMPOSITION DATABASE | 210 |
| CANDIDATE CULTURE MEDIUM COMPOSITION | 220 |
| CULTURE MEDIUM PANEL DATA | 230 |

# FIG. 6

```
        ┌─────────────┐
        │    START    │
        └─────────────┘
               │
               ▼
  ┌──────────────────────────────────┐
  │         SET REGION OF            │ ～ S100
  │ CULTURE MEDIUM COMPOSITION SPACE │
  └──────────────────────────────────┘
               │
               ▼
  ┌──────────────────────────────────┐
  │ LIST PLURALITY OF CELL CHARACTERISTICS │ ～ S110
  └──────────────────────────────────┘
               │
               ▼
  ┌──────────────────────────────────┐
  │       PREDICT CULTURE RESULT     │ ～ S120
  └──────────────────────────────────┘
               │
               ▼
  ┌──────────────────────────────────┐
  │         SEARCH AND LIST          │ ～ S130
  │   CULTURE MEDIUM COMPOSITIONS    │
  └──────────────────────────────────┘
               │
               ▼
  ┌──────────────────────────────────┐
  │  CONFIGURE CULTURE MEDIUM PANEL  │ ～ S140
  └──────────────────────────────────┘
               │
               ▼
  ┌──────────────────────────────────┐
  │       PRESENT INFORMATION        │ ～ S150
  │    ON CULTURE MEDIUM PANEL       │
  └──────────────────────────────────┘
               │
               ▼
        ┌─────────────┐
        │     END     │
        └─────────────┘
```

# FIG. 7

```
                    ┌─────────────┐
                    │    START    │
                    └──────┬──────┘
                           │
              ┌────────────▼────────────────┐
              │ CULTURE ENVIRONMENT INPUT STEP │─ S12
              └────────────┬────────────────┘
                           │
              ┌────────────▼────────────────┐
              │    BIOLOGICAL BEHAVIOR       │─ S14
              │   AMOUNT PREDICTION STEP     │
              └────────────┬────────────────┘
                           │
              ┌────────────▼────────────────┐
              │ CELL ENVIRONMENT CHANGE STEP │─ S16
              └────────────┬────────────────┘
                           │
                          S18
                    ◇─────────────◇
                    │   LAPSE OF   │  YES
                    │ PREDETERMINED TIME? │──────────► ┌──────────────┐
                    ◇─────────────◇                    │ OUTPUT STEP  │─ S24
                           │ NO                        └──────┬───────┘
                          S20                                 │
                    ◇─────────────◇                    ┌──────▼───────┐
               NO   │   SUPPLY OF  │                   │     END      │
              ◄─────│ CULTURE MEDIUM, NUTRIENTS,       └──────────────┘
                    │  AND SO ON?  │
                    ◇─────────────◇
                           │ YES
              ┌────────────▼────────────────┐
              │    CULTURE MEDIUM           │─ S22
              │ CONCENTRATION CHANGE STEP   │
              └─────────────────────────────┘
```

# FIG. 8

MACHINE LEARNING MODEL PREDICTION | MECHANISM-OF-ACTION MODEL PREDICTION

CELL DEATH MODEL

CULTURE MEDIUM MODEL

CELL METABOLISM MODEL

CONCENTRATION

〈INPUT〉
Ala
Arg
Asn
:
Val
NH₄⁺
NUMBER OF LIVING CELLS

CELL DEATH MODEL (PREDICTOR)

〈OUTPUT〉

⇒ VIABILITY

CELL DEATH

CONCENTRATION

GLUCOSE
AMINO ACIDS
VITAMINS
INORGANIC SUBSTANCES
O₂
NH₃
etc···

ABSORPTION RATE $F_A$

METABOLITE
METABOLIC REACTION

CELL GROWTH RATE $F_g$

ANTIBODY PRODUCTION RATE $F_p$

CONSUMPTION/PRODUCTION

EP 4 502 141 A1

# FIG. 9

# FIG. 10

$$V = \frac{d[S]}{dt} = \frac{Vmax[S]}{Km + [S]}$$

# FIG. 11

< CULTURE PERIOD AND MAIN CELL ACTIVITY>

CELL ACTIVITY

CULTURE DAYS

GROWTH

ANTIBODY
PRODUCTION

CELL DEATH
(CESSATION OF ACTIVITY)

GROWTH PHASE | ANTIBODY PRODUCTION PHASE | CELL DEATH PHASE (CESSATION OF ACTIVITY)

CELL METABOLISM MODEL

GROWTH MODEL

CULTURE MEDIUM → CELL GROWTH
ANTIBODY PRODUCTION

PRODUCTION MODEL

CULTURE MEDIUM → ANTIBODY PRODUCTION

CELL DEATH MODEL

CULTURE MEDIUM → CELL DEATH

EP 4 502 141 A1

# FIG. 12

<INPUT>                                    <OUTPUT>

Ala
Arg
Asn        CELL DEATH MODEL
...        (PREDICTOR)          ⇒  VIABILITY
Val
$NH_4^+$
NUMBER OF
LIVING CELLS

# FIG. 13

ANTIBODY YIELD

UNSUITABLE          UNSUITABLE
        CULTURE MEDIUM 1              CULTURE MEDIUM 2A    CULTURE MEDIUM 2B
                                                                        UNSUITABLE
                        CULTURE MEDIUM COMPOSITION

—— KNOWN CELL    ——— UNKNOWN CELL A    ---- UNKNOWN CELL B

# FIG. 14

FIG. 15

FIG. 16

## FIG. 17

| METHOD | Fa1 | Fa2 | Fa3 | Fa4 | Fa5 | Fa6 | Fb1 | Fb2 | Fb3 |
|---|---|---|---|---|---|---|---|---|---|
| (3) UNKNOWN PREDICTION | 20 (max) | 32 | 25 | 48 | 42 | 80 (max) | 66 (max) | 151 | 100 (max) |
| (2) KNOWN EXTREME VALUE | 20 (max) | 40 (max) | 50 (max) | 60 (max) | 70 (max) | 80 (max) | 66 (max) | 124 | 96 |
| (1A) UNIFORM A | 12.5 | 38 | 34 | 57 | 70 (max) | 80 (max) | 36 | 151 | 99 |
| (1B) UNIFORM B | 0 (down) | 38 | 49 (up) | 57 | 63 (down) | 80 (max) | 0 (down) | 160 (max) (up) | 99 |
| | The "(max)" indicates which of the methods (1) to (3) is successful in maximizing each of the cell characteristics Fa1 to Fb3 in the upper row. The "(up)" indicates a result of the method (1B) improved over that of the method (1A). The "(down)" indicates a result of the method (1B) lower than that of the method (1A). | | | | | | | | |

EP 4 502 141 A1

## INTERNATIONAL SEARCH REPORT

| International application No. |
| --- |
| **PCT/JP2023/011777** |

### A. CLASSIFICATION OF SUBJECT MATTER

*C12N 5/00*(2006.01)i; *C12M 1/00*(2006.01)i; *C12M 3/00*(2006.01)i
FI:   C12N5/00; C12M1/00 A; C12M3/00

According to International Patent Classification (IPC) or to both national classification and IPC

### B. FIELDS SEARCHED

Minimum documentation searched (classification system followed by classification symbols)

C12N5/00; C12M1/00; C12M3/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/MEDLINE/EMBASE/BIOSIS (STN), JSTPlus/JMEDPlus/JST7580 (JDreamIII), Japio-GPG/FX, PubMed

### C. DOCUMENTS CONSIDERED TO BE RELEVANT

| Category* | Citation of document, with indication, where appropriate, of the relevant passages | Relevant to claim No. |
| --- | --- | --- |
| X | WO 2021/166824 A1 (FUJIFILM CORP.) 26 August 2021 (2021-08-26)<br>     claims 1-2, paragraphs [0039], [0056], [0078]-[0080] | 1-3, 6-17 |
| Y | claims 1-2, paragraphs [0039], [0056], [0078]-[0080] | 1-17 |
| Y | WO 2015/182381 A1 (FUJIFILM CORP.) 03 December 2015 (2015-12-03)<br>     paragraph [0095] | 1-17 |
| Y | WO 2015/025508 A1 (FUJIFILM CORP.) 26 February 2015 (2015-02-26)<br>     claim 3 | 1-17 |
| Y | JP 2011-229410 A (NAGOYA UNIV.) 17 November 2011 (2011-11-17)<br>     claim 3 | 1-17 |
| A | WO 2005/021744 A1 (NATIONAL INST. OF ADVANCED INDUSTRIAL SCIENCE AND<br>TECHNOLOGY) 10 March 2005 (2005-03-10)<br>     entire text, all drawings | 1-17 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **05 June 2023** | **13 June 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

### INTERNATIONAL SEARCH REPORT
**Information on patent family members**

International application No.

**PCT/JP2023/011777**

| Patent document cited in search report | | | Publication date (day/month/year) | Patent family member(s) | | | Publication date (day/month/year) |
|---|---|---|---|---|---|---|---|
| WO | 2021/166824 | A1 | 26 August 2021 | US | 2022/0380717 | A1 | |
| | | | | claims 1-2, paragraphs [0048], [0071], [0099]-[0101] | | | |
| | | | | EP | 4108758 | A1 | |
| WO | 2015/182381 | A1 | 03 December 2015 | US | 2017/0073630 | A1 | |
| | | | | paragraph [0104] | | | |
| | | | | EP | 3150693 | A1 | |
| WO | 2015/025508 | A1 | 26 February 2015 | US | 2016/0163049 | A1 | |
| | | | | claim 3 | | | |
| JP | 2011-229410 | A | 17 November 2011 | (Family: none) | | | |
| WO | 2005/021744 | A1 | 10 March 2005 | US | 2006/0253258 | A1 | |
| | | | | entire text, all drawings | | | |
| | | | | EP | 1640453 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014503220 A

- WO 2021049044 A

**Non-patent literature cited in the description**

- **WURM, F.M.** ; **; WURM, M.J.** Cloning of CHO Cells, Productivity and Genetic Stability A Discussion.. *Processes*, 2017, vol. 5, 20, https://www.research-gate.net/publication/316314849_Cloning_of_-CHO_Cells_Productivity_ and_Genetic_Stability-A_Discussion/link/59410f0045851566e1beae32/-download

- **NEMHAUSER, GEORGE L.** ; **LAURENCE A. WOLSEY** ; **MARSHALL L. FISHER.** An analysis of approximations for maximizing submodular set functions - I. *Mathematical programming*, 1978, vol. 14 (1), 265-294, https://www.cs.toronto.edu/~eidan/pa-pers/submod-max.pdf
- **KRAUSE, ANDREAS et al.** Robust Submodular Observation Selection. *Journal of Machine Learning Research*, 2008, vol. 9 (12), https://jmlr.csail.mit.edu/papers/volume9/krause08b/krause08b.pdf